(19) Europäisches Patentamt
European Patent Office
Office européen des brevets

(11) Publication number: 0 024 365
B1

(12) # EUROPEAN PATENT SPECIFICATION

(45) Date of publication of patent specification: 21.11.84

(51) Int. Cl.$^3$: A 61 K 7/00

(21) Application number: 80200744.3

(22) Date of filing: 04.08.80

(54) **Cosmetic gel stick composition.**

(30) Priority: 15.08.79 US 66710
21.02.80 US 123238

(43) Date of publication of application:
04.03.81 Bulletin 81/09

(45) Publication of the grant of the patent:
21.11.84 Bulletin 84/47

(84) Designated Contracting States:
AT BE CH DE FR GB IT LI NL

(56) References cited:
EP-A-0 000 604
FR-A- 940 091
FR-A-1 123 007
FR-A-1 328 109
FR-A-1 598 998
FR-A-2 237 615
FR-A-2 248 021
FR-A-2 371 918
GB-A- 795 773
US-A-2 790 747
US-A-3 235 458

(73) Proprietor: THE PROCTER & GAMBLE COMPANY
301 East Sixth Street
Cincinnati Ohio 45202 (US)

(72) Inventor: Sampson, Ronald Lee
1580 Tremont Lane
Cincinnati Ohio 45222 (US)
Inventor: Shelton, David Lee
1600 Thomson Heights Drive
Cincinnati Ohio 34223 (US)

(74) Representative: Brooks, Maxim Courtney et al
Procter & Gamble (NTC) Limited Whitley Road
Longbenton
Newcastle-upon-Tyne NE12 9TS (GB)

EP 0 024 365 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to cosmetic compositions in the form of solid sticks. The compositions herein comprise a polyhydric alcohol, an ethylene oxide and/or propylene oxide condensation product and soap.

Attempts have been made to realize cosmetic sticks which deliver active ingredients to the skin such as deodorant materials via a vehicle which glides easily over the skin surface and which imparts a cooling sensation to the skin both during and after application. Soap/alcohol gels can provide such cosmetic benefits. Examples of soap gels are disclosed in US—A—2,732,327, US—A—2,857,315, US—A—2,900,306, and US—A—2,970,083.

While soap gels are old as evidenced by the above patents, such gels are not completely satisfactory. Generally, soap gels require considerable time to set up and often exhibit syneresis at elevated temperatures.

It is, therefore, an object of the present invention to provide cosmetic soap gel sticks which have reduced set-up times and syneresis while being aesthetically pleasing.

It is a further object of the present invention to provide cosmetic sticks which, when a perfume is present, have greater perfume scent on the skin than in the package.

It is a further object of the present invention to provide such cosmetic sticks which effectively deliver deodorant materials to the skin.

It has been surprisingly discovered that the above objectives can be realized by formulating a stick comprising the ingredients described below.

All percentages used herein are by weight of the total composition unless otherwise designated.

The present invention relates to cosmetic gel stick compositions comprising

(a) from 6% to 70% of an aliphatic, polyhydric alcohol having from 2 to 3 carbon atoms and from 2 to 3 hydroxyl groups;

(b) from 3% to 10% of soap selected from the sodium and potassium salts of $C_{14}$ to $C_{18}$ fatty acids and mixtures thereof;

and wherein the composition additionally comprises

(c) from 20% to 80% of a condensation product having the formula

$$H(C_2H_4O)_a(C_3H_6O)_bOR$$

wherein R is selected from hydrogen and hydrocarbon chains having from 2 to 20 carbon atoms, a and b are each from 0 to 35 and a + b is from 5 to 35.

The essential elements of the cosmetic gel sticks of the present invention as well as optional components, composition preparation, and composition use are discussed in detail below.

An essential component of the present cosmetic gel stick compositions is a polyhydric aliphatic alcohol containing 2 or 3 carbon atoms and from 2 to 3 hydroxyl groups. The polyhydric aliphatic alcohol component of the stick comprises from 6% to 70%, preferably from 15% to 70%, by weight of the composition.

Suitable polyhydric alcohols for use in the gel compositions herein include ethylene glycol, propylene glycol, trimethylene glycol, and glycerine. The most preferred polyol is propylene glycol.

Another essential component of the compositions herein is a gel forming agent. The gel forming agents used herein are the sodium and potassium salts (i.e. soaps) of fatty acids containing from 14 to 18 carbon atoms.

Soaps comprise from 3% to 10% by weight, preferably from 4% to 8% by weight of the composition. If soap concentrations lower than those specified are employed, the gels formed tend to be dimensionally unstable and tend to deform at summertime temperatures. If concentrations of soap above those specified are utilized, the gels formed tend to be too hard and do not exhibit desirable glide and application characteristics.

The fatty acid portion of the soap gel forming agents should be essentially pure saturated or unsaturated higher fatty acids having a $C_{14}$ to $C_{18}$ backbone. Suitable mixtures of such acids can be employed provided that such mixtures are free from significant proportions of other fatty acids of higher or lower chain length which substantially adversely affect or neutralize the desired gel forming effects.

Examples of fatty acids useful in synthesizing the gel forming agents herein include myristic, palmitic, stearic, oleic, linoleic, linolenic, margaric and the mixtures of such acids. Naturally occurring sources of such fatty acids include coconut oil, beef tallow, lanolin, fish oil, beeswax, palm oil, peanut oil, olive oil, cottonseed oil, soybean oil, corn oil, rapeseed oil, rosin acids, and greases. Conventional fractionation and/or hydrolysis techniques can be employed if necessary to obtain the requisite types of fatty acids from such materials.

Preferred fatty acid soap type gel forming agents include sodium stearate, sodium palmitate, potassium stearate, potassium palmitate and sodium myristate. The most preferred gel forming agent is sodium stearate.

Still another essential component of the present composition is an ethylene oxide and/or propylene oxide condensation product having the following formula:

$$H(C_2H_4O)_a(C_3H_6O)_bOR$$

wherein R is either hydrogen or a hydrocarbon chain having from 2 to 20 carbon atoms, preferably from 4 to 18, a and b are each from 0 to 35 and a + b is from 5 to 35.

Examples of such products are a condensate of about 14 moles of propylene oxide with one mole of butyl alcohol sold by Union Carbide under the name Fluid AP®; a polypropylene glycol having a molecular weight of 1200; a polyethylene glycol having a molecular weight of 420; a condensate of 20 moles of ethylene oxide and 5 moles of propylene oxide with one mole of cetyl alcohol; and a condensate of 15 moles of propylene oxide with one mole of stearyl alcohol. The preferred condensate is Fluid AP®.

The condensation product is present in the compositions of the present invention at a level of from 20% to 80%, preferably from 30% to 70% by weight of the composition.

The instant stick compositions can contain a variety of optional ingredients suitable for improving composition efficacy, stability, cosmetics and/or aesthetics. Such optional components include deodorant material, perfumes, dyes, pigments and coloring agents.

A highly preferred optional component of the instant composition is a material which helps retard alcohol evaporation and which acts as an antisyneresis agent. Especially preferred materials of this type are cellulose derivatives such as hydroxyalkylcelluloses. Especially preferred materials of this type are hydroxypropylcellulose compounds having the chemical formula:

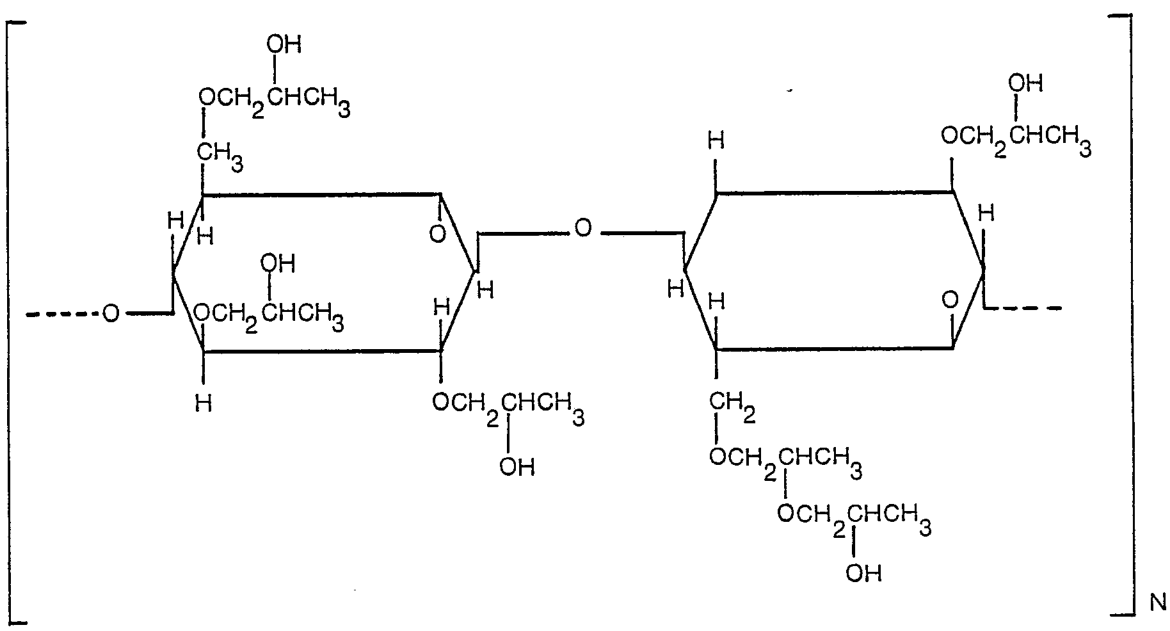

and wherein N is sufficiently large such that the total molecular weight of the material ranges from 60,000 to 1,000,000. Such materials are sold under the tradename of Klucel® by Hercules Incorporated. If present, such alcohol evaporation retarding agents and anti-syneresis agents comprise from 0.1% to 5.0% by weight of the composition.

Another optional ingredient of the instant compositions is a conventional deodorant material. Suitable deodorants include bacteriostatic quaternary ammonium compounds such as cetyl trimethylammonium bromide, cetyl pyridinium chloride, benzethonium chloride, diisobutylphenoxyethoxyethyl dimethyl benzyl ammonium chloride, N-alkylpyridinium chloride, N-cetyl pyridinium bromide, sodium N-lauroylsarcosine, sodium N-palmetoylsarcosine, N-lauroylsarcosine, N-hyristoylglycine, potassium N-lauroylsarcosine and stearyl trimethyl ammonium chloride. If present, deodorants generally comprise from 0.1% to 1.0% by weight of the composition.

Another optional component is a short chain monohydric alcohol in an amount from 0.1% to 50.0%, preferably from 1.0% to 40.0%. Suitable alcohols include methanol, ethanol, N-propanol and isopropanol. The preferred alcohol is ethanol.

Other optional ingredients such as perfumes, dyes, pigments and coloring agents, if present, comprise from 0.1% to 1.5% by weight of the compositions.

The gel sticks of the present invention are made by combining the ingredients in liquid form and pouring the mixture into a form having the desired shape. The present gel may be used as the gel portion of the antiperspirant sticks described and claimed in US—A—4,202,879. A preferred antiperspirant stick is where the present gel forms a shell around the antiperspirant core.

The gel sticks herein are used by the consumer by rubbing the stick on the area of the body where application is desired. In the case of a deodorant stick the stick is rubbed in the axilla area to apply the deodorant agent.

Examples I—VIII

Given below are examples of compositions within the scope of the present invention. In addition, compositions containing similar materials as well as one which does not contain an appropriate condensation product are shown.

| Formulae: | I | II | III | IV | V | VI | VII | VIII |
|---|---|---|---|---|---|---|---|---|
| Ethanol | 20.0 | 7.5 | — | 20.0 | — | 20.0 | 20.0 | — |
| Propylene Glycol | 15.5 | 27.9 | 24.0 | 25.5 | 24.0 | 25.5 | 70.0 | 24.0 |
| Fluid AP® 1 | 57.5 | 55.6 | — | — | — | — | — | 67.25 |
| Witconol APM® 2 | — | — | 67.25 | 47.5 | — | — | — | — |
| Carbitol® 3 | — | — | — | — | 67.25 | 47.5 | — | — |
| Sodium Stearate | 7.0 | 6.5 | 6.25 | 7.0 | 6.25 | 7.0 | 7.0 | 6.25 |
| Misc. | — | 2.5 | 2.5 | — | 2.5 | — | 3.0 | 2.5 |
| Set-up-time (Min.) | 2 | 9 | 35 | 45 | 38 | 17 | 30 | 8 |

1 Condensation product of one mole of butyl alcohol with 14 moles of propylene oxide supplied by Union Carbide Corporation.
2 Condensation product of one mole of myristyl alcohol with three moles of propylene oxide supplied by Witco Chemical Company.
3 Condensation product of one mole of butyl alcohol with two moles of ethylene oxide supplied by Union Carbide Corporation.

The above-mentioned compositions were made and poured into packages. The pour temperature of the compositions was 87.8°C (190°F) in each case. After pouring, the units were allowed to set at room temperature until gel set up was observed.

Gel Set-up Time (Definition):

The time necessary for the gel to set up to the point where there are no visible signs of exudation during trimming.

It is seen that the compositions containing Fluid AP® have significantly shorter set-up times than do the other compositions. The shorter times result in easier gel processing.

## Claims

1. A cosmetic gel stick composition comprising

(a) from 6% to 70% of an aliphatic, polyhydric alcohol having from 2 to 3 carbon atoms and from 2 to 3 hydroxyl groups;

(b) from 3% to 10% of soap selected from the sodium and potassium salts of $C_{14}$ to $C_{18}$ fatty acids and mixtures thereof;

characterized in that the composition additionally comprises

(c) from 20% to 80% of a condensation product having the formula

$$H(C_2H_4O)_a(C_3H_6O)_bOR$$

wherein R is selected from hydrogen and hydrocarbon chains having from 2 to 20 carbon atoms, a and b are each from 0 to 35 and a + b is from 5 to 35.

2. A stick composition according to Claim 1 characterized in that in addition, it contains from 0.1% to 1.0% of a deodorant material.

3. A stick composition according to Claim 1 or 2 characterized in that the level of polyhydric alcohol is from 15% to 70%, the level of soap is from 4% to 8% and the level of the condensation product is from 30% to 70%.

4. A stick composition according to any of Claims 1 to 3 characterized in that R is a hydrocarbon chain having from 4 to 18 carbon atoms.

5. A stick composition according to any of Claims 1 to 4 characterized in that in addition it contains a monohydric alcohol selected from methanol, ethanol, isopropanol and n-propanol.

6. A stick composition according to any of Claims 1 to 5 characterized in that the polyhydric alcohol is selected from ethylene glycol, propylene glycol, trimethylene glycol and glycerine.

7. A stick composition according to any of Claims 1 to 6 characterized in that the soap is selected from sodium stearate, sodium palmitate, sodium myristate, potassium stearate and potassium palmitate.

8. A stick composition according to any of Claims 1 to 7 characterized in that the polyhydric alcohol is propylene glycol.

9. A stick composition according to any of Claims 1 to 8 characterized in that the soap is sodium stearate.

10. A stick composition according to any of Claims 1 to 9 characterized in that the condensation product is the reaction product of one mole of butyl alcohol with about 14 moles of propylene oxide.

**Patentansprüche**

1. Eine gelierte Zusammensetzung für einen kosmetischen Stift, enthaltend

(a) 6% bis 70% eines aliphatischen, mehrwertigen Alkohols mit 2 bis 3 Kohlenstoffatomen und 2 bis 3 Hydroxylgruppen; und

(b) 3% bis 10% Seife, ausgewählt aus den Natrium- und Kaliumsalzen von $C_{14}$—$C_{18}$-Fettsäuren und Mischungen davon; dadurch gekennzeichnet, daß die Zusammensetzung zusätzlich

(c) 20% bis 80% eines Kondensationsproduktes mit der Formel

$$H(C_2H_4O)_a(C_3H_6O)_bOR$$

enthält, worin R aus Wasserstoff und Kohlenwasserstoffketten mit 2 bis 20 Kohlenstoffatomen ausgewählt ist, a und b jeweils 0 bis 35 sind und a + b 5 bis 35 ist.

2. Eine Zusammensetzung, für einen Stift, nach Anspruch 1, dadurch gekennzeichnet, daß sie zusätzlich 0,1% bis 1,0% eines Desodorantmaterials enthält.

3. Eine Zusammensetzung, für einen Stift, nach Anspruch 1 oder 2, dadurch gekennzeichnet, daß die Menge an mehrwertigem Alkohol 15% bis 70%, die Menge an Seife 4% bis 8% und die Menge des Kondensationsproduktes 30% bis 70% beträgt.

4. Eine Zusammensetzung, für einen Stift, nach einem der Ansprüche 1 bis 3, dadurch gekennzeichnet, daß R eine Kohlenwasserstoffkette mit 4 bis 18 Kohlenstoffatomen ist.

5. Eine Zusammensetzung, für einen Stift, nach einem der Ansprüche 1 bis 4, dadurch gekennzeichnet, daß sie zusätzlich einen einwertigen, aus Methanol, Ethanol, Isopropanol und n-Propanol ausgewählten Alkohol enthält.

6. Eine Zusammensetzung, für einen Stift, nach einem der Ansprüche 1 bis 5, dadurch gekennzeichnet, daß der mehrwertige Alkohol aus Ethylenglykol, Propylenglykol, Trimethylenglykol und Glycerin ausgewählt ist.

7. Eine Zusammensetzung, für einen Stift, nach einem der Ansprüche 1 bis 6, dadurch gekennzeichnet, daß die Seife aus Natriumstearat, Natriumpalmitat, Natriummyristat, Kaliumstearat und Kaliumpalmitat ausgewählt ist.

8. Eine Zusammensetzung, für einen Stift, nach einem der Ansprüche 1 bis 7, dadurch gekennzeichnet, daß der mehrwertige Alkohol Propylenglykol ist.

9. Eine Zusammensetzung, für einen Stift, nach einem der Ansprüche 1 bis 8, dadurch gekennzeichnet, daß die Seife Natriumstearat ist.

10. Eine Zusammensetzung, für einen Stift, nach einem der Ansprüche 1 bis 9, dadurch gekennzeichnet, daß das Kondensationsprodukt das Reaktionsprodukt von einem Mol Butylalkohol mit etwa 14 Mol Propylenoxid ist.

## Revendications

1. Composition gélifiée pour bâton cosmétique, comprenant

(a) de 6 à 70% d'un polyalcool aliphatique ayant 2 à 3 atomes de carbone et 2 à 3 groupes hydroxyle;

(b) de 3 à 10% d'un savon choisi parmi les sels de sodium et de potassium d'acides gras à 14 à 18 atomes de carbone et leurs mélanges;

caractérisée en ce que ladite composition comprend en outre

(c) de 20 à 80% d'un produit de condensation de formule

$$H(C_2H_4O)_a(C_3H_6O)_bOR$$

où R est choisi parmi l'hydrogène et les chaînes hydrocarbonées ayant 2 à 20 atomes de carbone, a et b valent chacun 0 à 35 et a + b est compris entre 5 et 35.

2. Composition pour bâton selon la revendication 1, caractérisée en ce que, en outre, elle contient de 0,1 à 1,0% d'une substance désodorisante.

3. Composition pour bâton selon la revendication 1 ou 2, caractérisée en ce que la concentration du polyalcool est de 15 à 70%, la concentration du savon est de 4 à 8% et la concentration du produit de condensation est de 30 à 70%.

4. Composition pour bâton selon l'une quelconque des revendications 1 à 3, caractérisée en ce que R est une chaîne hydrocarbonée ayant de 4 à 18 atomes de carbone.

5. Composition pour bâton selon l'une quelconque des revendications 1 à 4, caractérisée en ce qu'elle comprend en outre un monoalcool choisi parmi le méthanol, l'éthanol, l'isopropanol et le n-propanol.

6. Composition pour bâton selon l'une quelconque des revendications 1 à 5, caractérisée en ce que le polyalcool est choisi parmi l'éthylèneglycol, le propylèneglycol, le triméthylèneglycol et le glycérol.

7. Composition pour bâton selon l'une quelconque des revendications 1 à 6, caractérisée en ce que le savon est choisi parmi le stéarate de sodium, le palmitate de sodium, le myristate de sodium, le stéarate de potassium et le palmitate de potassium.

8. Composition pour bâton selon l'une quelconque des revendications 1 à 7, caractérisée en ce que le polyalcool est le propylèneglycol.

9. Composition pour bâton selon l'une quelconque des revendications 1 à 8, caractérisée en ce que le savon est le stéarate de sodium.

10. Composition pour bâton selon l'une quelconque des revendications 1 à 9, caractérisée en ce que le produit de condensation est le produit de la réaction d'une mole d'alcool butylique avec environ 14 moles d'oxyde de propylène.